# EUROPEAN PATENT APPLICATION

(11) **EP 3 895 714 A1**
(43) Date of publication of application: **20.10.2021**
(21) Application number: 19894607.1
(22) Date of filing: 20.11.2019
(51) Int. Cl.: A61K 35/74, A61K 9/00, A61P 29/00, A23L 33/135, A61K 8/99, A61Q 19/00

(54) **WEISSELLA BACTERIA-DERIVED NANOVESICLE AND USE THEREOF**

(30) Priority: 10.12.2018 KR 20180158621; 23.10.2019 KR 20190132136
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2019/015876
(87) International publication number: WO 2020/122448

(57) **Abstract**

The invention relates to vesicles derived from bacteria of the genus *Weissella* and a use thereof. The present inventors experimentally identified that vesicles derived from bacteria of the genus *Weissella* effectively suppress the secretion of inflammatory mediators from pathogenic vesicles inducing inflammation. Thus, it is expected that the vesicles derived from bacteria of the genus *Weissella* according to the present invention will be advantageously used for the purpose of developing a composition for preventing, alleviating, or treating inflammatory diseases.

## Description

### [Technical Field]

The present invention relates to nanovesicles derived from bacteria of the genus *Weissella* and a use thereof, and more particularly, to a composition for preventing, alleviating, or treating an inflammatory disease using nanovesicles derived from bacteria of the genus *Weissella.*

This application claims priority to and the benefit of Korean Patent Application Nos. 10-2018-0158621 and 10-2019-0132136 filed in the Korean Intellectual Property Office on December 10, 2018 and October 23, 2019, respectively, and all the contents disclosed in the specification and drawings of the applications are incorporated in this application.

### [Background Art]

Since the beginning of the 21st century, acute infectious diseases recognized as epidemic diseases in the past have become less important, whereas chronic inflammatory diseases accompanied by immune dysfunction caused by disharmony between humans and microbiomes have changed disease patterns as main diseases. Particularly, inflammatory diseases of the digestive system such as oral inflammatory diseases, chronic gastritis, gastric cancer, colitis and colorectal cancer, which are caused by westernized eating habits, are becoming a major problem for public health.

The development of the inflammatory diseases is accompanied by abnormalities in the immune function against external causative factors. The Th17 immune response, which secretes the interleukin (hereinafter, referred to as IL)-17 cytokine, is important for the immune response to causative factors derived from bacteria, and neutrophil inflammation due to the Th17 immune response occurs upon exposure to bacterial causative factors. Further, inflammatory mediators such as tumor necrosis factor-alpha (hereinafter, referred to as TNF-α), which is secreted by bacterial causative factors during the development of inflammation, play an important role in inflammation and cancer development. It has been recently reported that IL-6, which is secreted by bacterial causative factors, plays an important role in differentiation into Th17 cells, and chronic inflammation caused by the Th17 immune response is closely related to the development of chronic inflammatory diseases as well as cancer.

It is known that the number of microorganisms coexisting in the human body has reached 100 trillion, which is 10 times more than the number of human cells, and the number of microorganism genes is more than 100 times the number of human genes. A microbiota or microbiome refers to a microbial community including bacteria, archaea, and eukarya which are present in a given habitat, and it is known that the gut microbiota or microbiome plays an important role in human physiological phenomena and has a great influence on human health and disease through interaction with human cells.

Bacteria and archaea coexisting in our body secrete nanometer-sized vesicles in order to exchange information on genes, proteins, and the like with other cells. The mucosa forms a physical defense membrane through which particles having a size of 200 nanometers (nm) or more cannot pass, so that bacteria coexisting in the mucosa cannot pass through the mucosa, but vesicles derived from bacteria have a size of 100 nanometers or less and are absorbed into our bodies after relatively freely passing through epithelial cells via the mucosa. It has recently been revealed that pathogenic bacteria-derived vesicles absorbed in our body play an important role in the pathogenesis of metabolic diseases such as diabetes and obesity.

Bacteria of the genus *Weissella* are gram-positive cocci that secrete lactic acid, and are known as bacteria that coexist in the digestive system including the oral cavity. Particularly, *Weissella cibaria* is known to secrete hydrogen peroxide during symbiosis and thus inhibit the proliferation of *Fusobacterium nucleatum* related to the onset of periodontal disease, colitis, and colorectal cancer. However, there are no reports on therapeutic technology using extracellular vesicles derived from the bacteria of the genus *Weissella* yet.

Therefore, in the present invention, vesicles were first isolated from bacteria of the genus *Weissella,* and their characteristics were confirmed, thereby confirming that the vesicles can be used as a composition for preventing, alleviating or treating an inflammatory disease.

### [Disclosure]

### [Technical Problem]

As a result of conducting earnest research to solve the above-described conventional problems, the inventors confirmed that vesicles derived from bacteria of the genus *Weissella* effectively inhibit an inflammatory response generated by pathogenic vesicles, and based on this, the present invention was completed.

Thus, an object of the present invention is to provide a composition for preventing, alleviating, or treating an inflammatory disease, comprising vesicles derived from bacteria of the genus *Weissella* as an active ingredient.

However, a technical problem to be achieved by the present invention is not limited to the aforementioned problems, and the other problems that are not mentioned may be clearly understood by a person skilled in the art from the following description.

### [Technical Solution]

To achieve the object of the present invention as described above, the present invention provides a composition for preventing, alleviating, or treating an inflammatory disease, comprising vesicles derived from bacteria of the genus *Weissella* as an active ingredient.

The composition may comprise a pharmaceutical composition, a food composition, a cosmetic composition, and an inhalable composition.

Further, the present invention provides a method of preventing or treating an inflammatory disease, the method comprising a step of administering a composition comprising vesicles derived from bacteria of the genus *Weissella* as an active ingredient to a subject.

Further, the present invention provides a use of vesicles derived from bacteria of the genus *Weissella* for preventing or treating an inflammatory disease.

Further, the present invention provides a use of a composition comprising vesicles derived from bacteria of the genus *Weissella* as an active ingredient for preventing or treating an inflammatory disease.

Further, the present invention provides a use of vesicles derived from bacteria of the genus *Weissella* for preparing a drug used for an inflammatory disease.

As an exemplary embodiment of the present invention, the vesicles may be secreted from a *Weissella cibaria.*

As another exemplary embodiment of the present invention, the vesicles may have an average diameter of 10 to 200 nm.

As still another exemplary embodiment of the present invention, the vesicles may be secreted naturally or artificially from bacteria of the genus *Weissella.*

As yet another embodiment of the present invention, the artificially secreted vesicles may be secreted by performing a method such as heat treatment and pressure treatment on the bacteria.

As yet another embodiment of the present invention, the inflammatory disease may be one or more diseases selected from the group consisting of oral inflammatory diseases comprising gingivitis, periodontitis and oral cancer; gastric inflammatory diseases comprising gastritis and gastric cancer; colorectal inflammatory diseases comprising colitis, colon polyps and colorectal cancer; skin inflammatory diseases comprising atopic dermatitis and psoriasis; and respiratory inflammatory diseases comprising rhinitis, nasal polyps, asthma, chronic obstructive pulmonary disease and lung cancer.

### [Advantageous Effects]

The present inventors found that bacteria were not absorbed in the body, but vesicles derived from bacteria passed through the protective membrane of the mucosa, and were absorbed by the mucosal epithelial cells, systemically distributed, and excreted from the body through the kidneys, liver, and lungs. In addition, it was found that when *Weissella cibaria,* which is a species of bacteria of the genus *Weissella,* was cultured *ex vivo* and vesicles were separated and administered to inflammatory cells, the secretion of inflammatory mediators such as TNF-α from pathogenic vesicles were significantly suppressed, so that it is expected that the vesicles derived from bacteria of the genus *Weissella* according to the present invention will be advantageously used for a composition for prevention, alleviation, or treatment of the inflammatory diseases.

### [Description of Drawings]

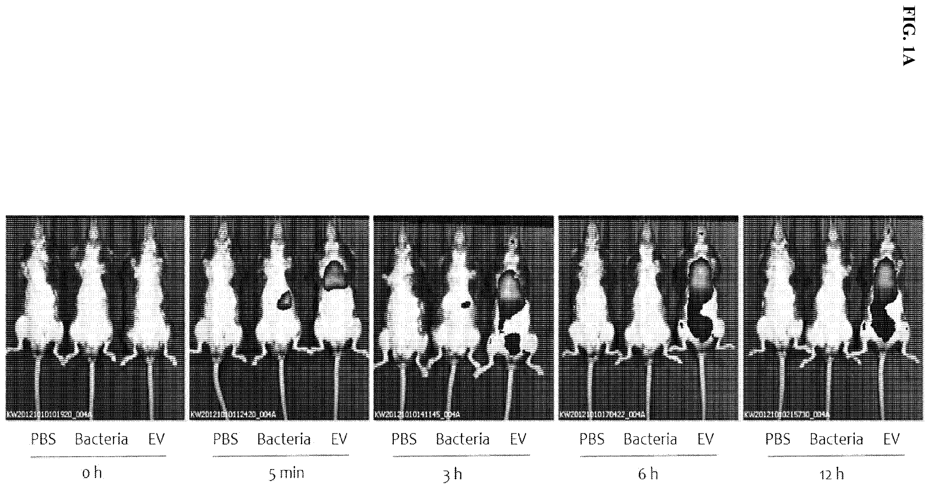

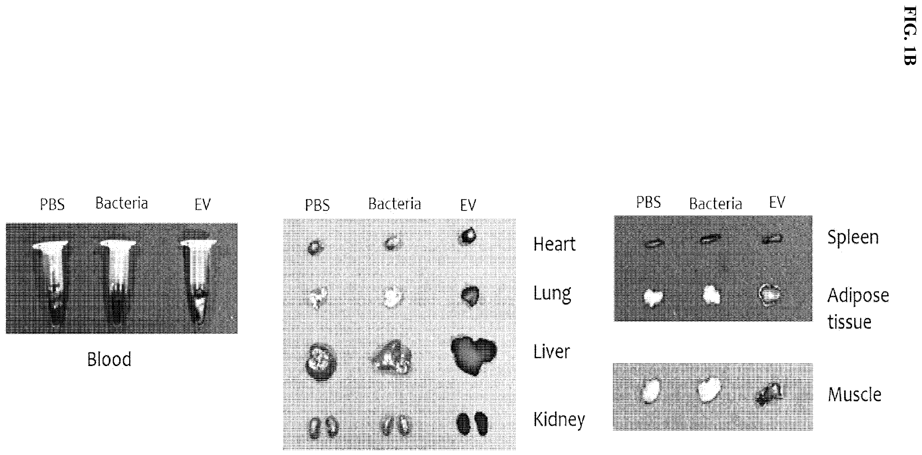

FIG. 1A is a series of photographs capturing distribution patterns of bacteria and bacteria-derived vesicles (EV) by time after the bacteria and the vesicles derived from bacteria were orally administered to mice, and FIG. 1B is a result of evaluating the in vivo distribution patterns of the bacteria and the vesicles by harvesting blood, kidneys, liver, and various organs at 12 hours after orally administering the bacteria and the vesicles.

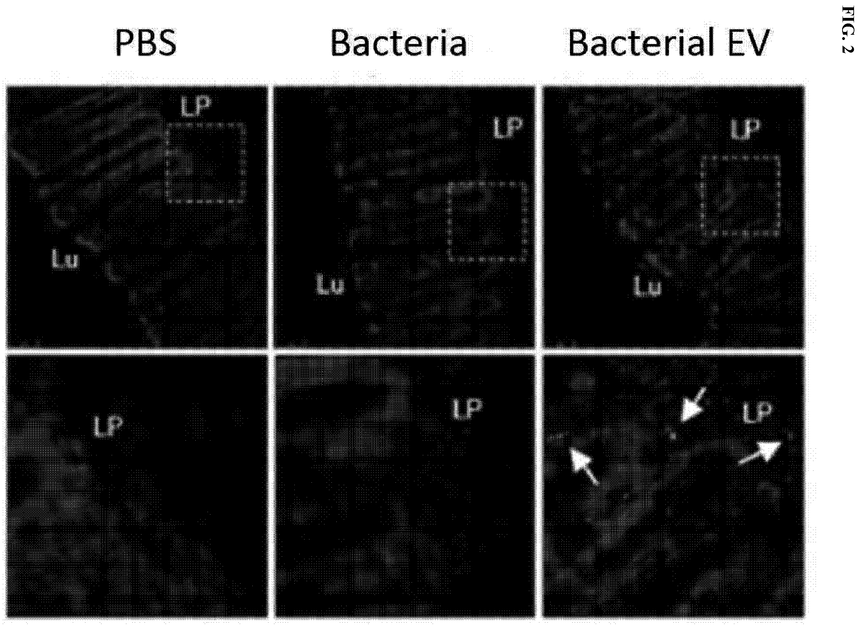

FIG. 2 is a view of evaluating whether bacteria and bacteria-derived vesicles (EV) are infiltrated into intestinal mucosal epithelial cells after administering the bacteria and bacteria-derived vesicles to the intestine of a mouse (Lu, gut lumen; LP, gut lamina propria).

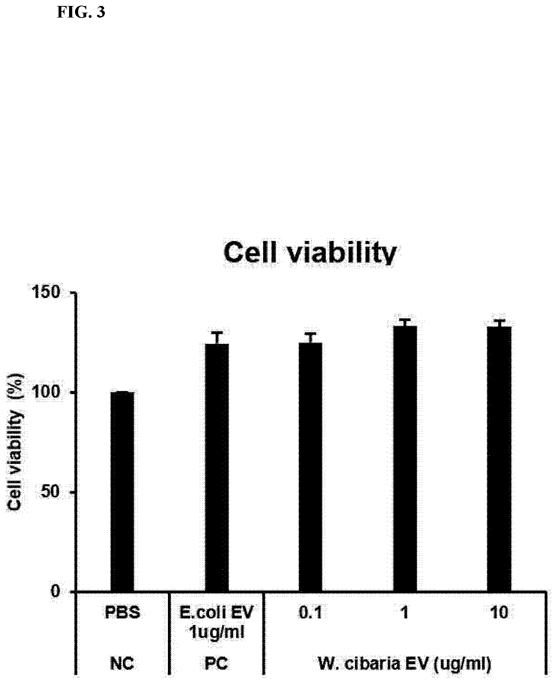

FIG. 3 is a result of evaluating apoptosis by treating microphages (Raw264.7 cells) with vesicles derived from *Weissella cibaria* in order to evaluate the apoptotic effects of vesicles derived from *Weissella cibaria* (EV, extracellular vesicle).

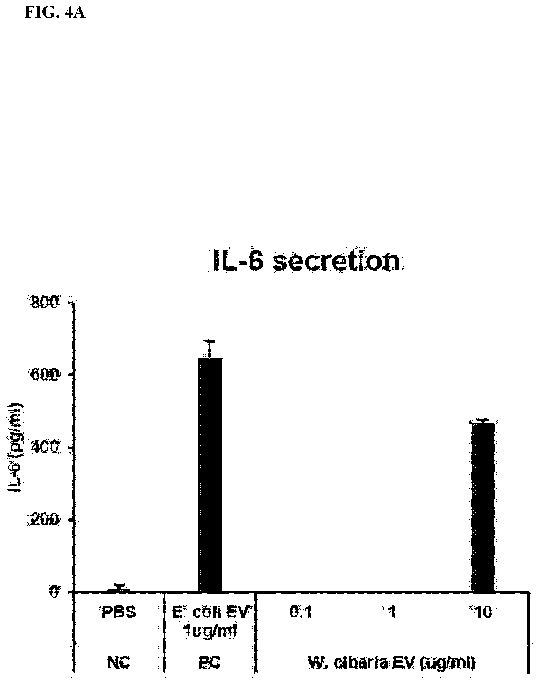

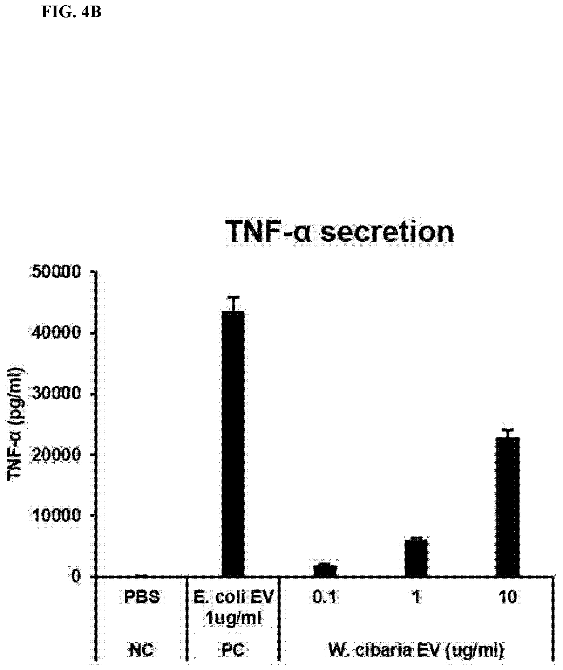

FIGS. 4A and 4B are results of comparing the secretion level of inflammatory mediators with that of *E. coli* EV which is a pathogenic vesicle by treating macrophages (Raw264.7 cells) with vesicles derived from *Weissella cibaria* in order to evaluate the inflammation induction effects of vesicles derived from *Weissella cibaria,* FIG. 4A compares the secretion levels of IL-6, and FIG. 4B compares the secretion levels of TNF-α (EV: extracellular vesicle).

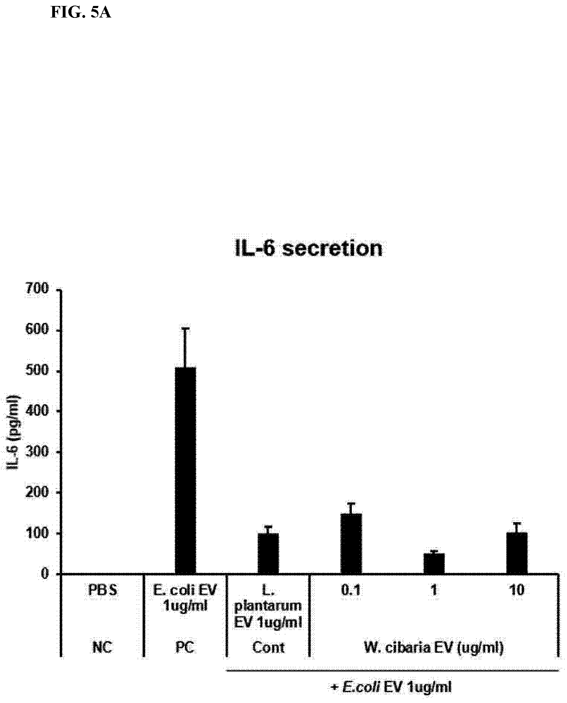

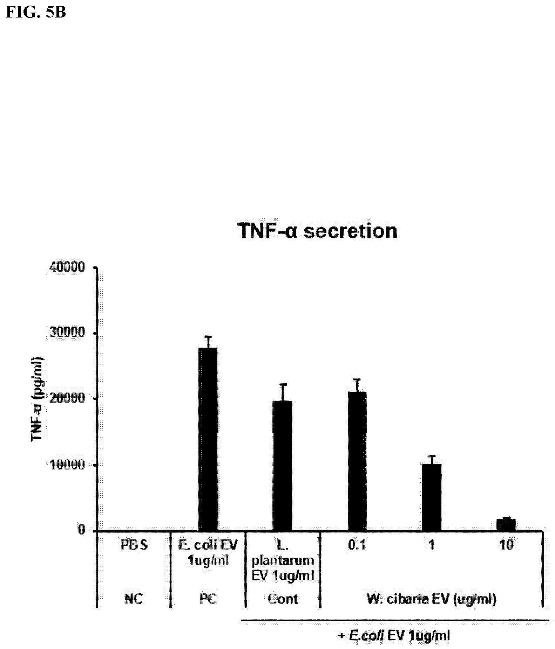

FIGS. 5A and 5B are results of evaluating the effect on the secretion of inflammation mediators by *E. coli* vesicles by pre-treating vesicles derived from *Weissella cibaria* before treatment of the *E. coli* EVs, which are pathogenic vesicles, to evaluate the anti-inflammatory effect of the vesicles derived from *Weissella cibaria,* in which FIG. 5A shows the comparison of the degree of IL-6 secretion, and FIG. 5B shows the comparison of the degree of TNF-α secretion (EV, extracellular vesicle).

### [Modes of the Invention]

The present invention relates to vesicles derived from bacteria of the genus *Weissella* and a use thereof.

The present inventors confirmed that the inflammatory response caused by pathogenic causative factors was efficiently suppressed by treating inflammatory cells with vesicles derived from *Weissella cibaria* bacterium before administering the pathogenic causative factors, thereby completing the present invention based on this.

Thus, the present invention provides a composition for preventing, alleviating, or treating an inflammatory disease, comprising vesicles derived from bacteria of the genus *Weissella* as an active ingredient.

The composition comprises a pharmaceutical composition, a food composition, a cosmetic composition, and an inhalable composition.

As another aspect of the present invention, the present invention provides a method of preventing or treating an inflammatory disease, the method comprising a step of administering a composition comprising vesicles derived from bacteria of the genus *Weissella* as an active ingredient to a subject.

As another aspect of the present invention, the present invention provides a use of vesicles derived from bacteria of the genus *Weissella* for preventing or treating an inflammatory disease.

As another aspect of the present invention, the present invention provides a use of a composition comprising vesicles derived from bacteria of the genus *Weissella* as an active ingredient for preventing or treating an inflammatory disease.

As another aspect of the present invention, the present invention provides a use of vesicles derived from bacteria of the genus *Weissella* for preparing a drug used for an inflammatory disease.

The term "nanovesicle" or "vesicle" as used herein refers to a structure consisting of a nano-sized membrane secreted from various bacteria. Vesicles derived from gram-positive bacteria such as *Weissella* include peptidoglycan and lipoteichoic acid, which are constituents of bacterial cell walls, and various low-molecular weight compounds in the vesicles, in addition to proteins and nucleic acids. In the present invention, nanovesicles or vesicles are secreted naturally from bacteria of the genus *Weissella* or produced artificially by performing heat treatment, pressure treatment, or the like on the bacteria, and have an average diameter of 10 to 200 nm.

The vesicles may be isolated from a culturing solution comprising bacteria of the genus *Weissella* by using one or more methods selected from the group consisting of centrifugation, ultra-high speed centrifugation, high pressure treatment, extrusion, sonication, cell lysis, homogenization, freezing-thawing, electroporation, mechanical decomposition, chemical treatment, filtration by a filter, gel filtration chromatography, free-flow electrophoresis, and capillary electrophoresis. Further, a process such as washing for removing impurities and concentration of obtained vesicles may be further included.

The term "inflammatory disease" as used herein refers to a disease which is caused by damage to skin or intestinal epithelial cells and consequent inflammation as a result of exposure to causative factors that induce inflammation, and includes cancer which occur as a result of inflammation. The inflammatory diseases comprises skin inflammatory diseases such as atopic dermatitis and psoriasis, which occur on the skin, respiratory inflammatory diseases such as rhinitis, nasal polyps, asthma, chronic obstructive pulmonary disease (COPD) and lung cancer, oral inflammatory diseases such as gingivitis, periodontitis and oral cancer, gastric inflammatory diseases such as gastritis, gastric ulcers and gastric cancer, and colorectal inflammatory diseases such as colitis, colon polyps and colorectal cancer, but the present invention is not limited thereto.

The term "prevention" as used herein refers to all actions that suppress an inflammatory disease, or delay the onset thereof via administration of the composition according to the present invention.

The term "treatment" as used herein refers to all actions that alleviate or beneficially change symptoms of an inflammatory disease via administration of composition according to the present invention.

The term "alleviation" used as used herein refers to all actions that at least reduce a parameter associated with a condition to be treated, for example, the degree of symptoms.

In one embodiment of the present invention, as a result of orally administering bacteria and vesicles derived from bacteria to mice and observing in vivo absorption, distribution, and excretion patterns of the bacteria and the vesicles, it was confirmed that, while the bacteria were not absorbed via the intestinal mucous membrane, the vesicles derived from bacteria were absorbed within 5 minutes after administration and systemically distributed, and excreted via the kidneys, liver, and the like (see Example 1).

In another exemplary embodiment of the present invention, it was evaluated whether bacteria and vesicles derived from bacteria directly administered to the intestines passed through the protective membrane of the intestinal mucosa, and it was confirmed that bacteria failed to pass through the protective membrane of the intestinal mucosa, whereas vesicles derived from bacteria passed through the protective membrane of the mucosa. (See Example 2).

In yet another exemplary embodiment of the present invention, inflammation induction effects of vesicles secreted from *Weissella cibaria* strains belonging to bacteria of the genus *Weissella* were evaluated by culturing the strains, and as a result of comparing the secretion levels of inflammatory mediators by treating macrophages with the vesicles derived from *Weissella cibaria* at various concentrations, and then treating the macrophages with the E. coli-derived vesicles, which are pathogenic vesicles, the ability of inflammatory mediators to be secreted was remarkably reduced by the vesicles derived from *Weissella cibaria* as compared to the secretion of IL-6 and TNF-α by E. coli-derived vesicles (see Example 4).

In yet another exemplary embodiment of the present invention, anti-inflammatory effects of vesicles derived from *Weissella cibaria* strains were evaluated. As a result of evaluating the secretion of inflammatory mediators after treating macrophages with vesicles derived from *Weissella cibaria* at various concentrations prior to treatment with E. coli-derived vesicles, which are pathogenic vesicles, it was confirmed the vesicles derived from *Weissella cibaria* efficiently suppressed the secretion of IL-6 and TNF-α by inflammation-inducing E. coli-derived vesicles (see Examples 5).

The pharmaceutical composition of the present invention may include a pharmaceutically acceptable carrier. The pharmaceutically acceptable carrier is typically used in formulation, and includes saline, sterile water, Ringer's solution, buffered saline, cyclodextrin, a dextrose solution, a maltodextrin solution, glycerol, ethanol, liposomes, and the like, but is not limited thereto, and may further include other typical additives such as an antioxidant and a buffer, if necessary. Further, the composition may be formulated into an injectable formulation, such as an aqueous solution, a suspension, and an emulsion, a pill, a capsule, a granule, or a tablet by additionally adding a diluent, a dispersant, a surfactant, a binder, a lubricant, and the like. With regard to suitable pharmaceutically acceptable carriers and formulations, the composition may be preferably formulated according to each ingredient by using the method disclosed in the Remington's literature. The pharmaceutical composition of the present invention is not particularly limited in formulation, but may be formulated into an injection, an inhalant, an external preparation for skin, an oral ingestion, or the like.

The pharmaceutical composition of the present invention may be orally administered or may be parenterally administered (for example, administered intravenously, subcutaneously, intradermally, intranasally or intratracheally) according to the target method, and the administration dose may vary depending on the patient's condition and body weight, severity of disease, drug form, and administration route and period, but may be appropriately selected by those of ordinary skill in the art.

The pharmaceutical composition according to the present invention is administered in a pharmaceutically effective amount. In the present invention, the pharmaceutically effective amount refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields. The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

Specifically, an effective amount of the pharmaceutical composition according to the present invention may vary depending on the age, sex, and body weight of a patient, and may be increased or decreased depending on the route of administration, severity of obesity, sex, body weight, age, and the like.

The inhalant composition of the present invention may be used by adding the active ingredient as it is to the inhalant, or may be used together with other ingredients, and may be appropriately used by a typical method. The mixing amount of active ingredient may be suitably determined depending on the purpose of use (for prevention or treatment).

The food composition of the present invention includes a health functional food composition. The food composition according to the present invention may be used by adding an active ingredient as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range.

Other ingredients are not particularly limited, except that the food composition of the present invention contains the active ingredient as an essential ingredient at the indicated ratio, and the food composition of the present invention may contain various flavorants, natural carbohydrates, and the like, like a typical beverage, as an additional ingredient. Examples of the above-described natural carbohydrate include common sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol. As the flavorant other than those described above, a natural flavorant (thaumatin, stevia extract (for example, rebaudioside A, glycyrrhizin and the like), and a synthetic flavorant (saccharin, aspartame and the like) may be advantageously used. The proportion of the natural carbohydrate may be appropriately determined by the choice of those of ordinary skill in the art.

The food composition of the present invention may contain various nutrients, vitamins, minerals (electrolytes), flavoring agents such as synthetic flavoring agents and natural flavoring agents, colorants and fillers (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in a carbonated beverage, or the like, in addition to the additives. These ingredients may be used either alone or in combinations thereof. The ratio of these additives may also be appropriately selected by those of ordinary skill in the art.

The cosmetic composition of the present invention may comprise not only vesicles derived from bacteria of the genus *Weissella,* but also ingredients commonly used in cosmetic compositions, and may comprise, for example, general adjuvants such as an antioxidant, a stabilizer, a solubilizing agent, vitamins, pigments, and herbs, and a carrier.

In addition, the composition of the present invention may further include, in addition to the vesicles derived from bacteria of the genus *Weissella,* a mixture of organic UV blocking agents that have long been used within a range that does not adversely affect a skin protective effect by reaction with vesicles derived from bacteria of the genus *Weissella.* The organic UV blocking agent may be one or more selected from the group consisting of glyceryl PABA, drometrizole trisiloxane, drometrizole, digalloyl trioleate, disodium phenyl dibenzimidazole tetrasulfonate, diethylhexyl butamido triazone, diethylamino hydroxy benzoyl hexyl benzoate, DEA-methoxycinnamate, a mixture of lawsone and dihydroxyacetone, methylenebis-benzotriazolyltetramethylbutylphenol, 4-methylbenzylidene camphor, menthyl anthranilate, benzophenone-3(oxybenzone), benzophenone-4, benzophenone-8(dioxybenzone), butylmethoxydibenzoylmethane, bisethylhexyloxyphenolmethoxyphenyltriazine, cinoxate, ethyldihydroxypropyl PABA, octocrylene, ethylhexyldimethyl PABA, ethylhexylmethoxycinnamate, ethylhexyl salicylate, ethylhexyl triazone, isoamyl-p-methoxycinnamate, polysilicon-15(dimethicodiethylbenzal malonate), terephthalylidene dicamphor sulfonic acid and salts thereof, TEA-salicylate, and para-aminobenzoic acid (PABA).

Examples of products to which the cosmetic composition of the present invention may be added include cosmetics such as astringents, skin softeners, nourishing toners, various creams, essences, packs, foundations, and the like, cleansings, face cleansers, soaps, treatments, beauty liquids, and the like. Particular preparations of the cosmetic composition of the present invention include a skin lotion, a skin softener, a skin toner, an astringent, a lotion, a milk lotion, a moisturizing lotion, a nourishing lotion, a massage cream, a nourishing cream, a moisturizing cream, a hand cream, an essence, a nourishing essence, a pack, a soap, a shampoo, a cleansing foam, a cleansing lotion, a cleansing cream, a body lotion, a body cleanser, an emulsion, a lipstick, a makeup base, a foundation, a press powder, a loose powder, an eye shadow, and the like.

Hereinafter, preferred Examples for helping the understanding of the present invention will be suggested. However, the following Examples are provided only to more easily understand the present invention, and the contents of the present invention are not limited by the following Examples.

### [Examples]

### Example 1. Analysis of in vivo Absorption, Distribution, and Excretion Patterns of Bacteria and Vesicles Derived from Bacteria

In order to evaluate whether bacteria and vesicles derived from bacteria were systemically absorbed through the gastrointestinal tract, an experiment was performed as follows. A dose of 50 µg of each of fluorescence-labeled bacteria and vesicles derived from bacteria in the stomach of a mouse was administered to the gastrointestinal tract, and fluorescence was measured after 0 minute, 5 minutes, 3 hours, 6 hours, and 12 hours. As a result of observing the entire image of the mouse, as illustrated in FIG. 1A, the bacteria were not systemically absorbed, but the vesicles derived from bacteria were systemically absorbed 5 minutes after administration, and fluorescence was strongly observed in the bladder 3 hours after administration, so that it could be seen that the vesicles were excreted to the urinary tract. Further, it could be seen that the vesicles were present in the body until 12 hours after administration (see FIG. 1A).

In order to evaluate the pattern in which the bacteria and the vesicles derived from the bacteria infiltrated into various organs after they were systemically absorbed, 50 µg of bacteria and vesicles derived from bacteria labeled with fluorescence were administered in the same manner as described above, and then the blood, heart, lungs, liver, kidneys, spleen, fat, and muscle were collected 12 hours after administration. As a result of observing fluorescence in the collected tissues, as illustrated in FIG. 1B, it could be seen that the vesicles derived from bacteria were distributed in the blood, heart, lungs, liver, kidneys, spleen, fat, and muscle but the bacteria were not absorbed (see FIG. 1B).

### Example 2. Evaluation of whether bacteria and vesicles derived from bacteria penetrate protective membrane of intestinal mucosa

In order to evaluate whether bacteria and bacteria-derived vesicles passed through the protective membrane of the intestinal mucosa to be infiltrated into intestinal tissue, after bacteria and bacteria-derived vesicles were directly administered to the intestines, infiltration into the intestinal tissue after passing through the protective membrane of the intestinal mucosa was evaluated by an immunohistochemistry method. In order to evaluate the presence of bacteria and vesicles in the intestinal mucosa, antibodies against the bacteria and the vesicles were prepared, attached to a green fluorescent protein (GFP) and used, and after staining with 4, 6-diamidino 2-phenylindole (DAPI), observed under a microscope.

As a result, it was confirmed that the bacteria cannot pass through the protective membrane of the intestinal mucosal, but the bacteria-derived vesicles infiltrate into the intestinal tissue through the intestinal mucosa (see FIG. 2).

### Example 3. Isolation of Vesicles from Weissella cibaria Culturing Solution

Based on the above examples, a *Weissella cibaria* strain were cultured, and then vesicles were isolated therefrom and characteristics of the isolated vesicles were analyzed. First, the *Weissella cibaria* strains were cultured in a de Man-Rogosa and Sharpe (MRS) medium in an incubator at 37°C until the absorbance (OD 600) became 1.0 to 1.5, and then sub-cultured in a Luria-Bertani (LB) medium. Subsequently, a culture supernatant including the strain was recovered and centrifuged at 10,000 x g and 4 °C for 20 minutes, and then the strain was removed and filtered through a 0.22 µm filter.

The filtered supernatant was concentrated to a volume of less than or equal to 50 ml through microfiltration by using a MasterFlex pump system (Cole-Parmer, US) with a 100 kDa Pellicon 2 Cassette filter membrane (Merck Millipore, US), and the concentrated supernatant was filtered once again with a 0.22-µm filter. Thereafter, proteins were quantified by using a BCA assay, and the following experiments were performed on the obtained vesicles.

### Example 4. Inflammation-inducing Effect of vesicles derived from Weissella cibaria

To examine an effect of vesicles derived from *Weissella cibaria (Weissella cibaria* EV) on the secretion of inflammatory mediators (IL-6 and TNF-α) in inflammatory cells, Raw 264.7 cells, which is a mouse macrophage line, were treated with vesicles derived from *Weissella cibaria* at various concentrations (0.1, 1, or 10 µg/ml), followed by apoptosis and ELISA.

More specifically, Raw 264.7 cells aliquoted at 5 x 10⁴ cells/well into a 48-well cell culture plate were treated with vesicles derived from *Weissella cibaria* at various concentrations, which were diluted with a DMEM (Dulbecos Modified Eagles Medium) serum-free medium, and the treated cells were cultured for 12 hours. Afterward, cell death was measured using EZ-CYTOX (Dogen, Korea), and the cell culture solution was collected in a 1.5-ml tube and centrifuged at 3000 x g for 5 minutes, and a supernatant was harvested and stored at -80 °C, followed by performing ELISA.

For ELISA, a capture antibody was diluted with phosphate buffered saline (PBS) and 50 µl aliquots thereof were dispensed into a 96-well polystyrene plate in accordance with a working concentration, and then allowed to react at 4 °C overnight. Subsequently, the sample was washed three times with 100 µl of a PBST (0.05% Tween-20-containing PBS) solution, and then an RD (1% bovine serum albumin (BSA)-containing PBS) solution was dispensed in 100 µl aliquots, followed by blocking at room temperature for 1 hour, and then the sample and a standard were dispensed in 50 µl aliquots in accordance with concentration and allowed to react at room temperature for 2 hours.

Then, the sample and the standard were washed three times with 100 µl of PBST, and then the detection antibody was diluted with RD, and the diluted solution was dispensed in 50 µl aliquots in accordance with a working concentration and allowed to react at room temperature for 2 hours. Thereafter, the sample and the standard were washed three times with 100 µl of PBST, and then streptavidin-horseradish peroxidase (HRP) (R&D Systems, USA) was diluted in RD to 1/40, and the diluted solution was dispensed in 50 µl aliquots and allowed to react at room temperature for 20 minutes. Lastly, the sample and the standard were washed three times with 100 µl of PBST, and then a 3,3',5,5'-tetramethylbenzidine (TMB) substrate (SurModics, USA) was dispensed in 50 µl aliquots, and then when color was developed after 5 minutes to 20 minutes, a 1M sulfuric acid solution was dispensed in 50 µl aliquots, thereby stopping the reaction, and absorbance at 450 nm was measured using a SpectraMax M3 microplate reader (Molecular Devices, USA).

As a result, as illustrated in FIG. 3, apoptosis due to the treatment with vesicles derived from *Weissella cibaria* was not observed (see FIG. 3). Further, as a result of evaluating the secretion pattern of inflammatory mediators in inflammatory cells, it was confirmed that the secretion of inflammatory mediators was much reduced upon treatment with vesicles derived from *Weissella cibaria* compared to upon treatment with E. coli-derived vesicles *(E. coli* EV 1 µg/ml), which are a positive control (see FIG. 4A and 4B).

### Example 15. Anti-inflammatory Effects of Vesicles derived from Weissella cibaria

In order to evaluate the anti-inflammatory effects of vesicles derived from *Weissella cibaria* based on the result of Example 4, after mouse macrophage cell lines were pre-treated with vesicles derived from *Weissella cibaria* at various concentrations (0.1, 1, and 10 µg/ml) for 12 hours, the cell lines were treated with 1 µg/ml of E. coli-derived vesicles, which are a pathogenic factor, and then the secretion of inflammatory cytokines was measured by ELISA after 12 hours.

As a result, it was found that the amount of IL-6, TNF-α secreted into inflammatory cells by *E. coli* EV stimulation during pre-treatment of vesicles derived from *Weissella cibaria* was remarkably suppressed (see FIG. 5A and 5B). In the case of IL-6, it was confirmed that an anti-inflammatory effect similar to when vesicles derived from *Lactobacillus plantarum,* which is a useful microbial control, were pretreated was exhibited (see FIG. 5A), and in the case of TNF-α, a it was confirmed that a significantly higher anti-inflammatory effect compared to when vesicles derived from *Lactobacillus plantarum,* which is a useful microbial control, were pretreated, is exhibited and the inhibitory effect is dependent on vesicles derived from *Weissella cibaria* treatment concentration (see FIG. 5B). This means that an inflammatory response induced by pathogenic inflammatory factors such as *E. coli* EVs can be effectively inhibited by the vesicles derived from *Weissella cibaria.*

The above-described description of the present invention is provided for illustrative purposes, and those of ordinary skill in the art to which the present invention pertains will understand that the present invention can be easily modified into other specific forms without changing the technical spirit or essential features of the present invention. Therefore, it should be understood that the above-described Examples are illustrative only in all aspects and are not restrictive.

### [Industrial Applicability]

The vesicles derived from bacteria of the genus *Weissella* according to the present invention may be absorbed by mucosal epithelial cells and distributed systemically by passing through the protective membrane of the mucosal, and may inhibit the secretion of inflammation mediators, such as IL-6 and TNF-α, by pathogenic vesicles. Therefore, the vesicles derived from bacteria of the genus *Weissella* are expected to be of great industrial value in that they can be effectively used in a composition for preventing, alleviating or treating an inflammatory disease.

## Claims

1. A pharmaceutical composition for preventing or treating an inflammatory disease, comprising vesicles derived from bacteria of the genus *Weissella* as an active ingredient.

2. The pharmaceutical composition of claim 1, wherein the vesicles derived from bacteria of the genus *Weissella* are secreted from a *Weissella cibaria.*

3. The pharmaceutical composition of claim 1, wherein the vesicles have an average diameter of 10 to 200 nm.

4. The pharmaceutical composition of claim 1, wherein the vesicles are secreted naturally or artificially from bacteria of the genus *Weissella.*

5. The pharmaceutical composition of claim 1, wherein the inflammatory disease is one or more diseases selected from the group consisting of oral inflammatory diseases comprising gingivitis, periodontitis and oral cancer; gastric inflammatory diseases comprising gastritis and gastric cancer; colorectal inflammatory diseases comprising colitis, colon polyps and colorectal cancer; skin inflammatory diseases comprising atopic dermatitis and psoriasis; and respiratory inflammatory diseases comprising rhinitis, nasal polyps, asthma, chronic obstructive pulmonary disease and lung cancer.

6. A food composition for preventing or alleviating an inflammatory disease, comprising vesicles derived from bacteria of the genus *Weissella* as an active ingredient.

7. The food composition of claim 6, wherein the vesicles derived from bacteria of the genus *Weissella* are secreted from a *Weissella cibaria.*

8. The food composition of claim 6, wherein the vesicles have an average diameter of 10 to 200 nm.

9. The food composition of claim 6, wherein the vesicles are secreted naturally or artificially from bacteria of the genus *Weissella.*

10. The food composition of claim 6, wherein the inflammatory disease is one or more diseases selected from the group consisting of oral inflammatory diseases comprising gingivitis, periodontitis and oral cancer; gastric inflammatory diseases comprising gastritis and gastric cancer; colorectal inflammatory diseases comprising colitis, colon polyps and colorectal cancer; skin inflammatory diseases comprising atopic dermatitis and psoriasis; and respiratory inflammatory diseases comprising rhinitis, nasal polyps, asthma, chronic obstructive pulmonary disease and lung cancer.

11. A cosmetic composition for preventing or alleviating an inflammatory disease, comprising vesicles derived from bacteria of the genus *Weissella* as an active ingredient.

12. The cosmetic composition of claim 11, wherein the vesicles are secreted naturally or artificially from bacteria of the genus *Weissella.*

13. An inhalable composition for preventing or treating an inflammatory disease, comprising vesicles derived from bacteria of the genus *Weissella* as an active ingredient.

14. A method of preventing or treating an inflammatory disease, the method comprising a step of administering a composition comprising vesicles derived from bacteria of the genus *Weissella* as an active ingredient to a subject.

15. A use of vesicles derived from bacteria of the genus *Weissella* for preparing a drug used for an inflammatory disease.
